# FASCICULE DE BREVET EUROPEEN

(11) **EP 4 140 535 B1**
(45) Date de publication et mention de la délivrance du brevet: **02.10.2024**
(21) Numéro de dépôt: 21315142.6
(22) Date de dépôt: 25.08.2021
(51) Int. Cl.: A61N 1/375, A61N 1/378, A61N 1/372, H02N 2/18, H10N 30/30

(54) **MODULE DE RÉCUPÉRATION D'ÉNERGIE À TRANSDUCTEUR PIÉZOÉLECTRIQUE EN DOUBLE PORTE-À-FAUX, NOTAMMENT POUR L'ALIMENTATION D'UNE CAPSULE CARDIAQUE AUTONOME LEADLESS**
ENERGIERÜCKGEWINNUNGSMODUL MIT PIEZOELEKTRISCHEM DOPPELAUSLEGER-WANDLER, INSBESONDERE FÜR DIE STROMVERSORGUNG EINER LEITUNGSLOSEN AUTONOMEN HERZKAPSEL
ENERGY RECOVERY MODULE WITH A DOUBLE CANTILEVER PIEZOELECTRIC TRANSDUCER, IN PARTICULAR FOR THE POWER SUPPLY OF A LEADLESS AUTONOMOUS CARDIAC CAPSULE

(43) Date de publication de la demande: 01.03.2023
(73) Titulaire: CAIRDAC, 92160 Antony (FR)
(72) Inventeur: REGNIER, Willy, 91160 Longjumeau (FR); NGUYEN-DINH, An, 37520 La Riche (FR); MAKDISSI, Alaa, 75011 Paris (FR); DOHIN, Julien, 92170 Vanves (FR); VASSAL, Steeven, 37200 Tours (FR); HOANG, Thien, 37000 Tours (FR)
(74) Mandataire: Dupuis-Latour, Dominique

(56) Documents cités:
- EP-A1- 3 693 056
- WO-A1-2014/116794
- US-A1- 2006 217 776
- US-A1- 2017 077 839
- US-A1- 2018 185 638

## Description

### CONTEXTE DE L'INVENTION

### Domaine de l'invention

L'invention concerne les récupérateurs d'énergie, également dénommés "harvesters" ou "scavengers", qui recueillent l'énergie mécanique résultant de mouvements divers qu'ils subissent et convertissent cette énergie mécanique en énergie électrique.

Elle concerne plus spécifiquement les récupérateurs de type dit "PEH" *(Piezoelectric Energy Harvester*), qui utilisent comme transducteur mécano-électrique une lame piézoélectrique oscillante couplée à une masse mobile inertielle.

L'invention sera décrite plus particulièrement dans une application de ces récupérateurs d'énergie à des dispositifs médicaux autonomes, notamment les dispositifs de type capsule implantable autonome, en particulier ceux de ces dispositifs qui sont destinés à être implantés dans une cavité cardiaque.

Cette application, si elle est particulièrement avantageuse, ne doit toutefois pas être considérée comme limitative de l'invention, dont les enseignements peuvent être appliqués à de nombreux autres types de dispositifs autonomes incorporant un récupérateur d'énergie de type PEH, que ces dispositifs soient implantables ou non, qu'il s'agisse de dispositifs médicaux ou non.

### Description de la technique antérieure

Dans le domaine des implants médicaux, les récents progrès en matière de miniaturisation de dispositifs actifs et les progrès des sciences du vivant permettent dorénavant le développement d'une grande variété de systèmes miniaturisés implantables totalement autonomes, à des fins de surveillance, de diagnostic ou de traitement. Ces dispositifs mettent en oeuvre des procédures d'implantation moins invasives, plus de confort, des performances accrues et souvent ouvrent l'accès à des nouveaux types de diagnostics et traitements.

Lorsqu'elle est appliquée au domaine des implants médicaux, l'invention concerne plus précisément ceux de ces implants qui incorporent un système d'autoalimentation comprenant un récupérateur d'énergie mécanique associé à un organe de stockage d'énergie intégré tel qu'une batterie rechargeable ou une capacité à hautes performances.

En effet, l'un des aspects critiques de ces dispositifs miniaturisés est celui de l'autonomie électrique. La durée de vie d'un tel implant étant d'environ 8 à 10 ans, compte tenu des très faibles dimensions il n'est pas possible d'utiliser une batterie conventionnelle, même à forte densité.

Le récupérateur d'énergie pallie cet inconvénient en recueillant l'énergie mécanique résultant des mouvements divers subis par le corps du dispositif implanté. Ces mouvements peuvent avoir pour origine un certain nombre de phénomènes se produisant par exemple au rythme des battements cardiaques tels que les secousses périodiques de la paroi sur laquelle est ancré l'implant, les vibrations des tissus cardiaques liées entre autres aux fermetures et ouvertures des valves cardiaques, ou encore les variations de débit du flux sanguin dans le milieu environnant, qui sollicitent l'implant et le font osciller au rythmé des variations de débit. L'énergie mécanique recueillie par le récupérateur est convertie en énergie électrique (tension ou courant) au moyen d'un transducteur mécanoélectrique approprié, pour assurer l'alimentation des divers circuits et capteurs du dispositif et la recharge de l'organe de stockage d'énergie. Ce système d'alimentation permet au dispositif de fonctionner en parfaite autonomie électrique pendant toute sa durée de vie.

Cette technique de récupération d'énergie est particulièrement bien adaptée à l'alimentation des capsules autonomes implantées dépourvues de toute liaison physique à un dispositif distant. Ces capsules sont dénommées pour cette raison "capsules *leadless",* pour les distinguer des électrodes ou capteurs disposés à l'extrémité distale d'une sonde *(lead)* parcourue sur toute sa longueur par un ou plusieurs conducteurs reliés à un générateur connecté à l'extrémité opposée, proximale.

L'invention n'est toutefois pas limitée à un type particulier de capsule, ni même d'implant leadless, et elle est applicable indifféremment à de nombreux autres types de dispositifs médicaux implantables, quelle que soit leur destination fonctionnelle, cardiaque ou autre, médicale ou non. Dans le cas d'une application cardiaque, la capsule leadless surveille en continu le rythme du patient et délivre si nécessaire au coeur des impulsions électriques de stimulation, de resynchronisation et/ou de défibrillation en cas de troubles du rythme détecté par la capsule. La capsule comprend par ailleurs divers circuits électroniques, capteurs, etc. ainsi que des moyens émetteurs/récepteurs de communication sans fil pour l'échange de données à distance, le tout étant intégré dans un corps de très petites dimensions qui puisse être implanté dans des sites difficilement accessibles ou laissant peu d'espace, tels que l'apex du ventricule, la paroi interne de l'oreillette, etc.

Le WO 2019/001829 A1 (Cairdac) décrit un exemple d'une telle capsule leadless intracardiaque. Une capsule leadless intracardiaque est aussi décrit dans le EP3693056A1.

L'invention concerne plus précisément les capsules ou dispositifs implantables analogues dont le récupérateur d'énergie est du type PEH, c'est-à-dire utilisant un transducteur piézoélectrique ou "PZT" *(PieZoelectric Transducer*) et un ensemble pendulaire inertiel soumis aux sollicitations externes décrites plus haut. L'ensemble pendulaire inertiel comprend une masse mobile logée dans le corps de la capsule, dite "masse sismique" ou "masse inertielle", qui est entraînée au gré des mouvements de la capsule soumise en permanence aux diverses sollicitations externes décrites plus haut. Après chacune de ces sollicitations, la masse inertielle, qui est couplée à un élément élastiquement déformable, oscille à une fréquence propre d'oscillation libre.

L'énergie mécanique de l'oscillation est convertie en énergie électrique par un transducteur mécanoélectrique produisant un signal électrique. Ce transducteur mécanoélectrique peut en particulier être un PZT sollicité de façon cyclique en flexion de manière à engendrer au sein du matériau qui le constitue des charges électriques qui sont récupérées en surface du composant pour être utilisées par le système d'autoalimentation de la capsule leadless. Le PZT se présente le plus souvent sous la forme d'une lame encastrée à l'une de ses extrémités et couplée à la masse inertielle à son autre extrémité, qui est libre.

Le signal électrique en sortie du transducteur est délivré à un circuit de gestion de l'alimentation de la capsule, qui redresse et régule le signal électrique pour délivrer en sortie une tension ou un courant continus stabilisés permettant d'assurer l'alimentation des divers circuits électroniques et capteurs de la capsule, ainsi que la recharge de l'organe de stockage d'énergie.

Un tel récupérateur d'énergie de type PEH est notamment décrit dans le US 3,456,134 A (Ko) et dans le WO 2019/001829 A1 précité. Un récupérateur d'énergie est aussi décrit dans le US2006217776A1.

On notera que le terme de "lame" doit être entendu dans son sens le plus large, à savoir une bande mince et plate de forme allongée, étant entendu que la forme de cette bande n'est pas nécessairement rectangulaire ni son épaisseur constante (comme dans la description du mode de réalisation particulier qui sera donnée plus bas). Le terme de "lame" au sens de la présente invention recouvre ainsi des éléments pouvant présenter une largeur et/ou une épaisseur qui ne sont pas constantes en direction longitudinale, ainsi qu'éventuellement une déformabilité pouvant aller au-delà d'un unique degré de liberté en flexion.

Le problème de l'invention réside dans la recherche d'une plus grande compacité dimensionnelle du PEH, tout en préservant ses capacités fonctionnelles de production d'énergie en quantité suffisante pour assurer l'alimentation des circuits de la capsule autonome, et la recharge de la batterie tampon intégrée.

En particulier, si l'on souhaite réaliser des capsules leadless qui puissent être implantées dans l'oreillette, la forme tubulaire allongée des capsules conventionnelles n'est pas adaptée, du fait du volume beaucoup plus réduit de la cavité atriale par rapport au ventricule, et de la nécessité en outre d'implanter la capsule sur une paroi latérale de l'oreillette : une capsule allongée devrait alors être implantée avec sa plus grande dimension plus ou moins perpendiculaire à la veine cave supérieure, ce qui concrètement n'est pas possible avec cette voie d'accès. A l'opposé, pour une capsule leadless ventriculaire implantée au fond du ventricule droit, l'apex se situe dans le prolongement de la veine cave supérieure et de la valve tricuspide, donc ne pose pas de difficulté particulière d'implantation, et de plus la forme allongée de la capsule n'est pas gênante compte tenu du plus grand volume de la cavité ventriculaire.

Il en serait de même pour une capsule épicardique, c'est-à-dire implantée à la surface extérieure du myocarde, où il serait souhaitable de disposer d'un élément de forme plutôt plate qu'allongée.

En particulier, si l'on souhaite mettre en place une stimulation de type double chambre il est nécessaire de disposer d'électrodes au niveau de l'oreillette pour assurer les fonctions requises de détection et/ou de stimulation atriale.

L'implantation d'une sonde conventionnelle reliée à un boîtier de générateur distant et munie d'électrodes au niveau de l'oreillette et du ventricule ne pose pas de difficulté particulière. En revanche, avec la technique léadless il est nécessaire d'implanter une première capsule dans le ventricule et une seconde capsule dans l'oreillette, les deux capsules étant munies de moyens de communication mutuelle pour assurer les fonctions requises de la stimulation double chambre.

La réduction de la longueur de la capsule est donc un préalable indispensable à la réalisation d'une capsule leadless auriculaire implantable.

Mais si l'on alimente une telle capsule par un récupérateur de type PEH, la présence de la lame PZT, qui doit être assez longue pour permettre un débattement suffisant pour produire assez de charges électriques afin d'alimenter convenablement les circuits de la capsule, implique pour la capsule un facteur de forme en longueur, qui est celui typiquement rencontré sur la quasi-totalité des capsules décrites par l'état de la technique, avec une forme de tube allongé.

Ce problème de la recherche d'une compacité accrue n'est toutefois pas spécifique aux capsules leadless cardiaques, auriculaires ou autres, mais se pose chaque fois que l'on veut miniaturiser un système PEH, quelle que soit l'application envisagée.

De façon générale, dans une optique de miniaturisation poussée et d'intégration du design d'un PEH, il est souhaitable de réduire le volume globalement nécessaire à l'oscillation de la lame PZT, qui est un volume perdu, dans lequel il n'est pas possible de placer des composants électriques, électroniques ou mécaniques, ou qui permettrait de disposer d'une batterie un peu plus volumineuse procurant une capacité accrue.

Dans cette même optique, il serait également souhaitable qu'un même élément de structure puisse assurer plusieurs fonctions, afin de réduire le volume global du système, en faciliter l'industrialisation et réduire le coût de fabrication.

### RÉSUMÉ DE L'INVENTION

Pour résoudre les différents problèmes et atteindre les buts exposés ci-dessus, l'invention propose un module de récupération d'énergie de type PEH comprenant, de manière en elle-même connue, notamment d'après le WO 2019/001829 A1 précité, un ensemble pendulaire soumis à des sollicitations externes appliquées au module, l'ensemble pendulaire comprenant un transducteur piézoélectrique élastiquement déformable en flexion avec une extrémité encastrée et une extrémité libre couplée à une masse inertielle, le transducteur piézoélectrique étant apte à convertir une énergie mécanique produite par des oscillations de l'ensemble pendulaire en un signal électrique oscillant recueilli par des électrodes de surface du transducteur piézoélectrique. L'invention est définie par les revendications indépendantes 1 et 13.

De façon caractéristique, selon un *aspect* de l'invention : le transducteur piézoélectrique comprend deux lames piézoélectriques coplanaires agencées côte-à-côte, chacune des lames piézoélectriques comprenant une branche externe et une branche interne attenantes agencées côte-à-côte et formées d'un seule pièce ; la branche externe de chaque lame piézoélectrique a une extrémité proximale encastrée et une extrémité distale libre ; et la branche interne de chaque lame piézoélectrique a une extrémité proximale libre et supportant la masse inertielle, et une extrémité distale libre et réunie à l'extrémité distale de la branche externe attenante par une jonction commune. De la sorte, les oscillations de l'ensemble pendulaire produisent des déplacements respectifs des deux branches internes dans un premier sens et, simultanément, des deux branches externes dans un second sens opposé.

Selon diverses formes de réalisation avantageuses :
- il est prévu des électrodes de surface sur l'une au moins des branches externe et interne de chaque lame piézoélectrique, et le module comprend en outre un étage redresseur recevant sur des entrées respectives les charges recueillies par les électrodes de surface de chaque lame piézoélectrique et délivrant en sortie une signal d'alimentation redressé ;
- il est prévu des électrodes de surface sur l'une et l'autre des branches externe et interne de chaque lame piézoélectrique, les électrodes de surface respectives des branches interne et externe étant aptes à recueillir des charges de polarités opposées au cours d'une même déformation du transducteur piézoélectrique ;
- les électrodes de surface recueillant des charges de même polarité sont directement reliées ensemble et couplées à une même entrée respective de l'étage redresseur ;
- les lames piézoélectriques sont des lames bimorphes comprenant une âme centrale conductrice formant électrode commune et, pour chaque branche interne et/ou externe, des électrodes de surface sur une face supérieure et sur une face inférieure ;
- l'étage redresseur comprend une pluralité de circuits redresseurs associés à des paires d'électrodes respectives délivrant des charges de polarités opposées, et les sorties de la pluralité de circuits redresseurs sont directement reliées ensemble ;
- les branches externe et interne de chaque lame piézoélectrique sont parallèles et/ou rectilignes et/ou sont de même longueur pour les deux lames piézoélectriques ;
- les branches interne et externe sont de longueurs différentes pour les deux lames piézoélectriques respectives ;
- les branches internes respectives des deux lames piézoélectriques sont partiellement ou totalement confondues en une branche unique ;
- la lame piézoélectrique porte à son extrémité distale libre une masselotte intermédiaire à l'endroit de la jonction ;
- les deux lames piézoélectriques portent une masselotte intermédiaire commune à l'endroit de la jonction ; et/ou
- les deux lames piézoélectriques portent chacune indépendamment une masselotte propre à l'endroit de la jonction.

L'invention a également pour objet un dispositif autonome logeant, dans un corps de dispositif : un ensemble électronique ; un module de récupération d'énergie tel que ci-dessus, produisant en sortie un signal électrique oscillant ; un circuit de gestion d'alimentation, apte à redresser et réguler le signal électrique oscillant produit par le module de récupération d'énergie, pour délivrer en sortie une tension ou un courant continus d'alimentation stabilisés ; et un organe de stockage d'énergie pour l'alimentation de l'ensemble électronique. Ladite tension ou ledit courant continus stabilisés sont délivrés par le circuit de gestion d'alimentation servent à l'alimentation de l'ensemble électronique et/ou à la recharge de l'organe de stockage d'énergie du dispositif autonome.

Le dispositif autonome peut en particulier être un dispositif médical actif de type capsule autonome implantable comprenant un corps de capsule avec un élément d'ancrage à une paroi d'un organe d'un patient, les sollicitations externes auxquelles est soumis l'ensemble pendulaire du module de récupération d'énergie étant des sollicitations appliquées au corps de capsule sous l'effet de mouvements de ladite paroi et/ou de variations de débit d'un flux dans le milieu environnant.

### DESCRIPTION SOMMAIRE DES DESSINS

On va maintenant décrire un exemple de réalisation de la présente invention en référence aux dessins annexés, où les mêmes références désignent d'une figure à l'autre des éléments identiques ou fonctionnellement semblables.
La Figure 1 illustre des dispositifs médicaux de type capsule leadless dans leur environnement, avec divers exemples de sites d'implantation dans, sur ou à proximité du coeur d'un patient.
La Figure 2 illustre une capsule leadless implantée au fond du ventricule droit d'un patient.
La Figure 3 montre isolément un ensemble pendulaire de type connu, avec un PZT en forme de lame allongée encastrée à une extrémité et supportant une masse inertielle à son extrémité opposée.
La Figure 4 présente sous forme schématique les principaux blocs fonctionnels constitutifs d'une capsule leadless.
La Figure 5 est une vue en coupe montrant, avec une échelle des épaisseurs volontairement exagérée, la structure des différentes couches d'un PZT bimorphe utilisable avec la présente invention.
La Figure 6 est une vue en plan d'un PZT selon l'invention, sous forme de deux lames agencées côte à côte avec pour chacune deux branches parallèles interne et externe.
La Figure 7 est homologue de la Figure 6, pour une variante avec une courbure non arrondie à la jonction entre les deux branches interne et externe.
La Figure 8 est une vue perspective du PZT de la Figure 6, muni d'électrodes de surface sur chacune des faces inférieure et supérieure de chaque branche externe.
La Figure 9 est homologue de la Figure 8, pour une variante où les branches externes et également internes sont munies d'électrodes de surface.
La Figure 10 est un schéma électrique simplifié montrant la manière de coupler les différentes électrodes de surface du PZT de la Figure 9 à des redresseurs afin de débiter en sortie un courant de recharge d'une batterie tampon.
La Figure 11 présente une série de chronogrammes montrant les variations instantanées de l'accélération du PZT et les divers signaux électriques recueillis par les électrodes avant et après redressement et couplage mutuel.
La Figure 12 illustre une variante du PZT de l'invention, dans laquelle les deux branches internes sont réunies en une unique branche commune aux deux lames.
La Figure 13 est une vue en perspective montrant le PZT de la Figure 9 équipé d'une masse inertielle d'extrémité et fixé à une monture qui définit les encastrements des lames et assure en outre une fonction de limitation de course.
La Figure 14 est une vue en coupe schématique de l'ensemble de la Figure 13, avec le PZT mobile en position centrale.
La Figure 15 est homologue de la Figure 14, en configuration de déformation maximale du PZT avec butée de la masse inertielle contre une surface interne de la monture.
La Figure 16 est homologue de la Figure 15, pour une variante dans laquelle la masse inertielle est de forme externe plane et la surface homologue interne de butée de la monture est inclinée.
La Figure 17 est une vue en coupe transversale de la monture de la Figure 16, montrant le contact plan en butée de la masse inertielle contre la surface interne homologue de la monture.
La Figure 18 est homologue de la Figure 17, pour une variante dans laquelle le contact en butée est un contact linéaire axial.
La Figure 19 est homologue de la Figure 17, pour une autre variante dans laquelle le contact en butée est un contact surfacique-cylindrique.
La Figure 20 est homologue de la Figure 15, pour un contact de la masse inertielle contre une face interne de la monture revêtue d'une couche de polymère ou de silicone.
La Figure 21 est homologue de la Figure 16, pour un contact de masse inertielle contre une face interne de la monture revêtue d'une couche de polymère ou de silicone.
La Figure 22 est homologue de la Figure 14, pour une variante dans laquelle le montage de la masse inertielle à la lame est un montage soudé, sans collage.
La Figure 23 est homologue de la Figure 13, pour un mode de réalisation dans lequel une masselotte intermédiaire est ajoutée sur une face du PZT, au niveau de la jonction entre les branches internes et externes.
La Figure 24 est homologue de la Figure 23, pour une variante où des masselottes sont ajoutées sur chacune des faces supérieure et inférieure du PZT.
La Figure 25 est une vue en coupe homologue de la Figure 15, pour la variante de la Figure 24.
La Figure 26 est homologue de la Figure 23, pour une variante dans laquelle des masselottes distinctes sont ajoutées sur une face du PZT, séparément pour chacune des lames du PZT.
La Figure 27 est homologue de la Figure 26, pour une variante dans laquelle les masselottes sont ajoutées sur chacune des faces supérieure et inférieure du PZT.
La Figure 28 est homologue de la Figure 27, pour une variante dans laquelle les deux lames du PZT ontdes longueurs respectives différentes.

### DESCRIPTION DÉTAILLÉE DE MODES DE RÉALISATION PRÉFÉRENTIELS DE L'INVENTION

On va maintenant décrire un exemple de réalisation du dispositif de l'invention, dans une application à une capsule implantable autonome destinée à être implantée dans une cavité cardiaque.

Comme on l'a indiqué plus haut, cette - application particulière n'est donnée qu'à titre d'exemple de réalisation et n'est pas limitative de l'invention, dont les enseignements peuvent être appliqués à de nombreux autre types de dispositifs autonomes incorporant un récupérateur d'énergie de type PEH, que ces dispositifs soient implantables ou non, qu'il s'agisse de dispositifs médicaux ou non.

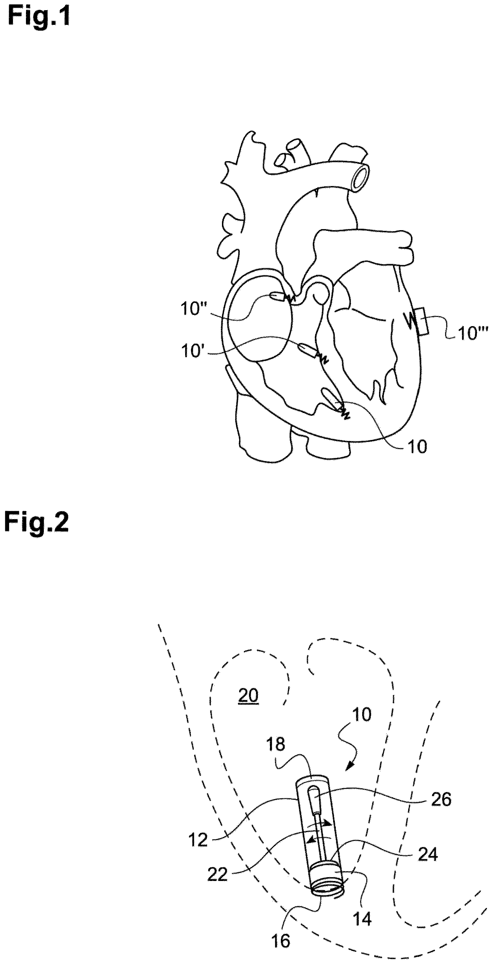

Sur la Figure 1, on a représenté diverses possibilités de sites d'implantation d'un dispositif de type leadless, dans une application à la stimulation cardiaque. Ainsi, la capsule 10 est implantée à l'intérieur d'une cavité du myocarde (implant endocavitaire), par exemple à l'apex du ventricule droit. En variante, la capsule peut être également implantée sur le septum interventriculaire droit, comme en 10', ou encore sur une paroi auriculaire, comme illustré en 10". Le dispositif peut également être une capsule épicardique placée sur une région externe du myocarde, comme illustré en 10‴.

Dans chaque cas, la capsule leadless est fixée à la paroi cardiaque au moyen d'un système d'ancrage saillant pénétrant dans le tissu cardiaque pour le maintien sur le site d'implantation. D'autres systèmes d'ancrage sont utilisables, et ne modifient en aucune façon la mise en oeuvre de la présente invention.

La capsule 10 se présente sous forme extérieure d'un implant avec un corps tubulaire allongé 12 enfermant les divers circuits électroniques et d'alimentation de la capsule ainsi qu'un récupérateur d'énergie à ensemble pendulaire. Les dimensions typiques des capsules connues sont un diamètre de l'ordre de 6 mm pour une longueur d'environ 25 à 40 mm.

Le corps tubulaire 12 comporte à son extrémité avant (distale) 14 un élément d'ancrage saillant, par exemple une vis hélicoïdale 16 pour maintenir la capsule sur le site d'implantation. D'autres systèmes d'ancrage sont utilisables, et ne modifient en aucune façon la mise en oeuvre de la présente invention. L'extrémité opposée (proximale) 18 de la capsule 10 est une extrémité libre, qui est seulement pourvue de moyens (non représentés) de liaison temporaire à un cathéter-guide ou autre accessoire d'implantation utilisé pour la mise en place ou l'explantation de la capsule, qui est ensuite désolidarisé de cette dernière.

Dans l'exemple illustré Figure 2, la capsule leadless 10 est un implant endocavitaire implanté à l'intérieur d'une cavité 20 du myocarde, par exemple à l'apex du ventricule droit. En variante, toujours dans une application à la stimulation cardiaque, la capsule peut être également implantée sur le septum interventriculaire ou sur une paroi auriculaire, ou encore être une capsule épicardique placée sur une région externe du myocarde, ces différents modes d'implantation ne modifiant en aucune façon la mise en oeuvre de la présente invention. Pour assurer les fonctions de détection/stimulation, une électrode (non représentée) en contact avec le tissu cardiaque au site d'implantation assure le recueil des potentiels de dépolarisation cardiaque et/ou l'application d'impulsions de stimulation. Dans certaines formes de réalisation, la fonction de cette électrode peut être assurée par la vis d'ancrage 16, qui est alors une vis active, électriquement conductrice et reliée au circuit de détection/stimulation de la capsule.

La capsule leadless 10 est par ailleurs dotée d'un module récupérateur d'énergie dit "PEH", comprenant un ensemble pendulaire inertiel qui oscille à l'intérieur de la capsule au gré des diverses sollicitations externes auxquelles la capsule est soumise. Ces sollicitations peuvent notamment résulter : des mouvements de la paroi à laquelle est ancrée la capsule, qui sont transmis au corps tubulaire 12 par la vis d'ancrage 16 ; et/ou des variations du débit du flux sanguin dans le milieu environnant la capsule, qui produisent des oscillations du corps tubulaire 12 au rythme des battements cardiaques ; et/ou des vibrations diverses transmises par les tissus cardiaques.

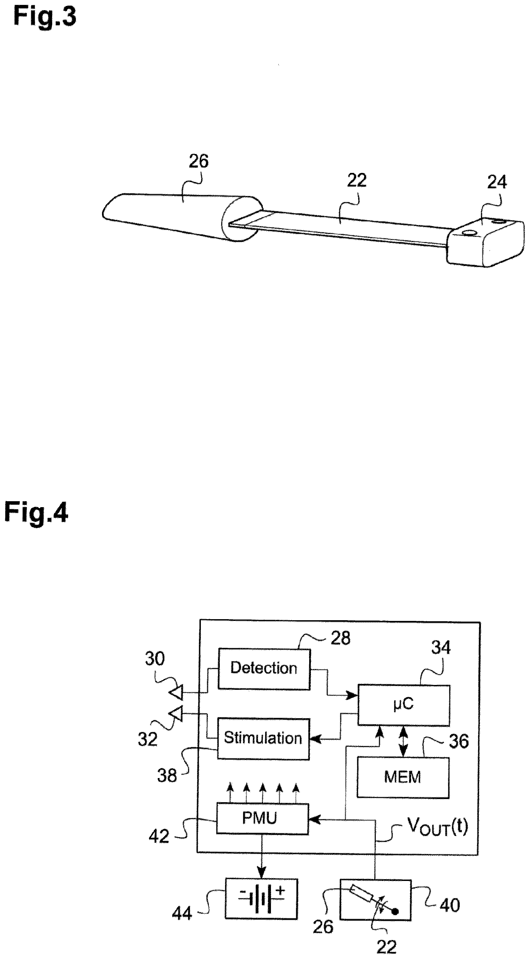

L'ensemble pendulaire, illustré isolément Figure 3, est constitué d'une lame piézoélectrique 22 encastrée en 24 à l'une de ses extrémités et dont l'extrémité opposée, libre, est couplée à une masse inertielle mobile 26. La lame piézoélectrique 22 est une lame flexible élastiquement déformable qui constitue avec la masse inertielle 26 un système pendulaire de type masse-ressort. Du fait de son inertie, la masse 26 soumet la lame 22 à une déformation de type vibratoire de part et d'autre d'une position neutre ou non déformée correspondant à une position stable de repos en l'absence de toute sollicitation. Les dimensions typiques minimales des lames PZT des dispositifs connus de ce type sont de l'ordre de 25 mm de long pour une largeur de 5 mm environ.

De fait, pour ce qui est de son comportement mécanique, cet ensemble peut être assimilé à une structure de type "poutre encastrée-libre", présentant une fréquence d'oscillation propre qui est ici la fréquence sur laquelle oscille le système masse-ressort. On notera que cette fréquence d'oscillation propre, typiquement de l'ordre de quelques dizaines de hertz, est notablement supérieure à la fréquence des sollicitations cycliques externes qui correspondent à la fréquence des battements cardiaques (quelques hertz tout au plus). Ainsi, à chaque contraction cardiaque la masse inertielle (ou autre organe mécanique fonctionnellement analogue) sera sollicitée avec une amplitude plus ou moins importante, puis le système pendulaire oscillera plusieurs fois avec des amplitudes décroissantes (rebonds caractéristiques d'une oscillation périodique amortie), et enfin se stabilisera jusqu'au battement cardiaque suivant, où le cycle sollicitation/oscillations se reproduira de façon comparable.

La lame 22 assure en outre, par effet piézoélectrique, une fonction de transducteur mécanoélectrique permettant de convertir en charges électriques la contrainte mécanique de flexion qui lui est appliquée. Ces charges sont collectées par des électrodes à la surface de la lame de manière à produire un signal électrique qui, après redressement, stabilisation et filtrage, alimentera les divers circuits électroniques de la capsule.

La Figure 4 est un synoptique des divers circuits électriques et électroniques intégrés à la capsule leadless, présenté sous forme de blocs fonctionnels.

Le bloc 28 désigne un circuit de détection de l'onde de dépolarisation cardiaque, qui est relié à une électrode de cathode 30 en contact avec le tissu cardiaque et à une électrode d'anode 32 associée, par exemple une électrode annulaire formée sur le corps tubulaire de la capsule. Le bloc de détection 28 comprend des filtres et des moyens de traitement analogique et/ou numérique du signal recueilli. Le signal ainsi traité est appliqué à l'entrée d'un microcalculateur 34 associé à une mémoire 36. L'ensemble électronique comporte également un circuit de stimulation 38 opérant sous le contrôle du microcalculateur 34 pour délivrer au système d'électrodes 30, 32 des impulsions de stimulation du myocarde.

Il est par ailleurs prévu un circuit récupérateur d'énergie ou PEH 40, constitué par l'ensemble pendulaire formé par la lame piézoélectrique 22 et la masse inertielle 26 décrits plus haut en référence aux Figures 2 et 3. Comme la lame piézoélectrique 22 assure aussi une fonction de transducteur mécano-électrique, elle convertit en charges électriques les sollicitations mécaniques subies et produit un signal électrique variable V_{OUT}(t), qui est un signal alternatif oscillant à la fréquence d'oscillation libre de l'ensemble pendulaire lame 22/masse 26, et au rythme des battements successifs du myocarde auquel la capsule est couplée.

Le signal électrique variable V_{OUT}(t) est délivré à un circuit de gestion de l'énergie ou PMU 42. Le PMU 42 redresse et régule le signal V_{OUT}(t) de manière à produire en sortie une tension ou un courant continus stabilisés servant à l'alimentation des divers circuits électroniques et à la recharge de la batterie intégrée 44.

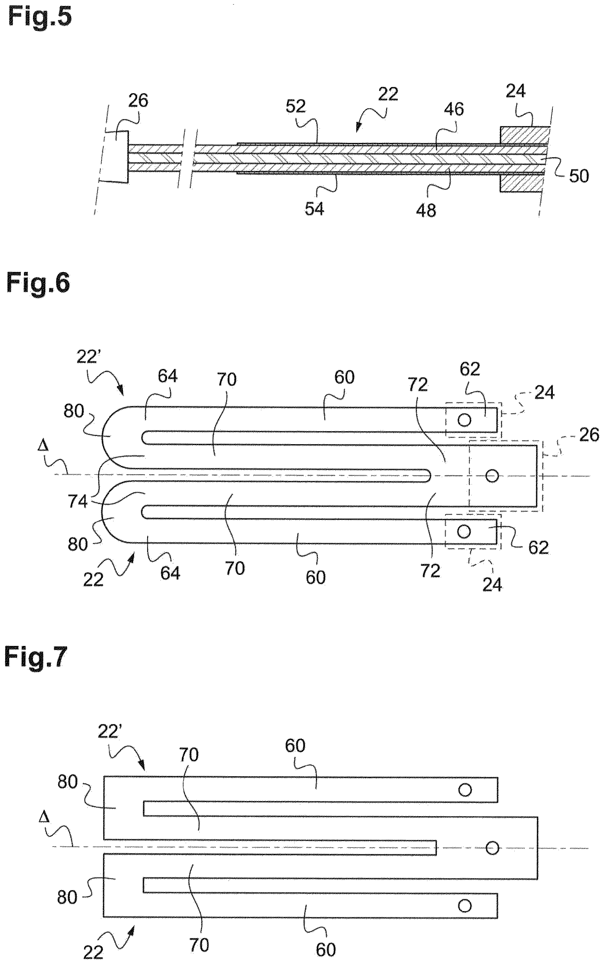

D'autre part, la lame est avantageusement une lame de type bimorphe, c'est-à-dire capable de générer de l'énergie sur ses deux faces lorsqu'elle est soumise à une déformation. Ces propriétés de transduction sont typiques d'un matériau piézoélectrique tel que les céramiques PZT ou les monocristaux de type PMN-PT, titanate de baryum ou niobate de lithium. La Figure 5 illustre de façon schématique, avec une échelle des épaisseurs volontairement exagérée, la structure des différentes couches d'une telle lame PZT bimorphe.

La lame 22 bimorphe comprend deux couches 46, 48 de matériau ,céramique PZT déposées sur chacune des faces opposées d'une âme centrale ou *shim* 50 en matériau conducteur (ou en variante en un matériau isolant, avec des ponts de contact permettant de shunter les électrodes internes des couches piézoélectriques). Cette structure bimorphe correspond en fait à l'association de deux structures unimorphes placées dos-à-dos, avec mise en commun de l'âme supportant le matériau PZT. Si l'âme 50 est en matériau conducteur, il est possible de recueillir les charges produites par la déformation du matériau PZT aussi bien entre l'âme conductrice 50 et une électrode de surface 52, 54 de l'un et/ou de l'autre des PZT 46, 48, qu'entre les deux électrodes de surface 52, 54 des faces opposées de la lame, indépendamment de l'âme centrale.

La Figure 6 est une vue en plan d'un PZT selon l'invention, considéré isolément avant réalisation des encastrements et mise en place de la masse inertielle.

De façon caractéristique de l'invention, ce PZT est formé de deux lames piézoélectriques 22, 22' coplanaires agencées côte à côte, de part et d'autre d'un axe central Δ correspondant à la direction de plus grande longueur du PZT.

Chacune des deux lames 22, 22' présente une forme repliée avec deux branches 60, 70 attenantes, agencées côte à côte et formées d'une seule pièce. La branche externe 60 a son extrémité proximale 62 encastrée (encastrement 24) et son extrémité distale 64 libre, tandis que la branche interne 70 a son extrémité proximale 72 qui est libre en déplacement et qui supporte la masse inertielle 26, et son extrémité distale 74 qui est libre (ici et dans la suite, le côté "proximal" sera entendu comme étant celui qui est le plus proche de l'encastrement de la lame, et le côté "distal" le côté opposé, le plus éloigné de l'encastrement). L'extrémité distale 64 de la branche externe 60 et l'extrémité distale 74 de la branche interne 70 sont réunies ensemble par une jonction commune 80.

Le PZT constitué des deux lames 22, 22' avec pour chacune les branches internes et externes que l'on vient de décrire peut être notamment réalisé par découpe au laser dans une unique pièce de matériau.

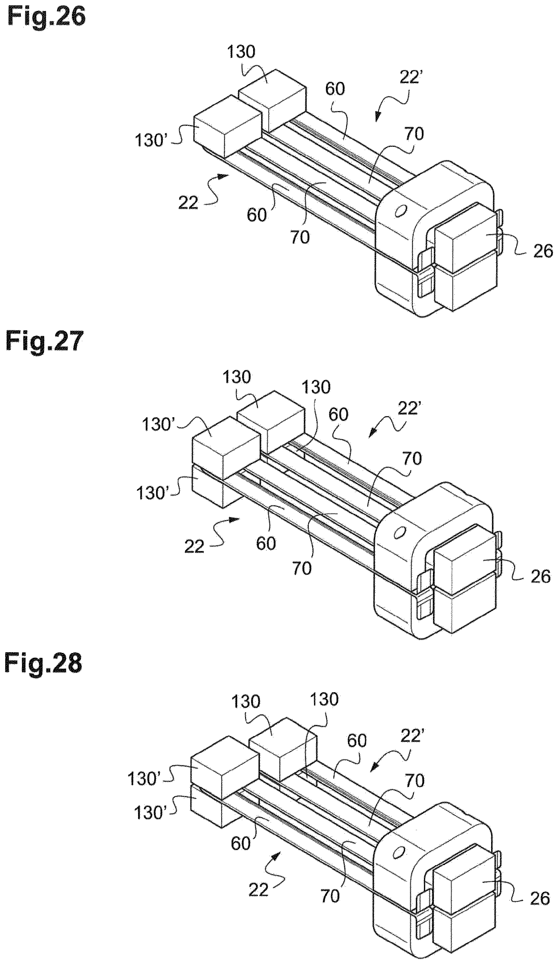

Les deux lames 22, 22' peuvent être symétriques (comme illustré) ou non, de même longueur (comme illustré) ou non (la Figure 28 montre en particulier un exemple où les deux lames 22, 22' ont des longueurs différentes).

Pour chaque lame, les branches externe et interne 60, 70 peuvent être approximativement parallèles- (comme illustré) ou non, avoir des bords parallèles (comme illustré) ou non, et être de même longueur et/ou largeur (comme illustré) ou non.

Comme cela est décrit dans la demande EP 20315317.6 du 24.06.2020 pour *"Module de récupération d'énergie à transducteur piézoélectrique, notamment pour la recharge optimisée de la batterie d'un dispositif médical implantable tel qu'une capsule cardiaque autonome leadless",* afin de renforcer la fiabilité des lames PZT 22, 22', il est possible de donner à celles-ci, sur tout ou partie de leur longueur, une forme en plan trapézoïdale avec une décroissance (linéaire ou exponentielle) de la largeur pour mieux répartir les contraintes le long de la lame, ces contraintes étant plus fortes à proximité et au niveau de l'encastrement 24, et nulles au niveau de la masse inertielle 26. De plus, la forme trapézoïdale permet d'ajuster la fréquence de résonance de l'ensemble en fonction de la géométrie du trapèze, tout en maximisant l'amplitude de déplacement de la masse du fait que l'extrémité libre est plus étroite que l'extrémité encastrée.

Avec la configuration que l'on vient de décrire, chacune des deux lames 22, 22' présente un double porte-à-faux correspondant à chacune des deux branches externe et interne 60, 70, à savoir : un premier porte-à-faux pour la branche externe 60 entre l'encastrement 24 à l'extrémité proximale 62 et l'extrémité distale 64 qui est libre d'osciller ; et un second porte-à-faux pour la branche interne 70 entre l'extrémité distale 74 liée à l'extrémité distale 64 (mobile) de la branche externe 60 et l'extrémité proximale 72 portant la masse inertielle 26.

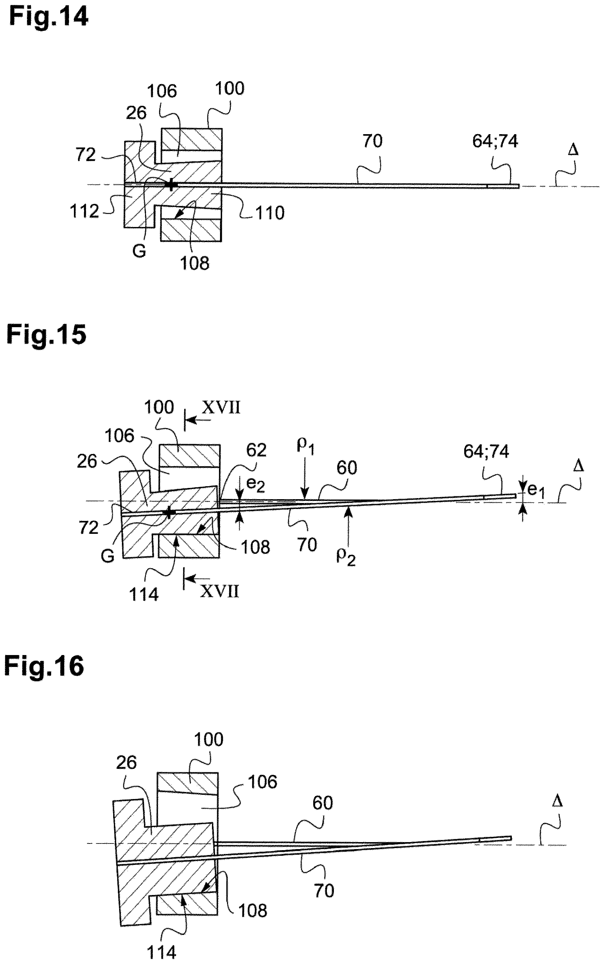

Les oscillations de l'ensemble pendulaire constitué par l'encastrement 24, la lame 22 et la masse inertielle 26 produisent des déplacements respectifs des deux branches 60, 70 dans des sens respectifs opposés (on pourra se référer plus loin à la Figure 15 qui montre, en élévation et en coupe, les flexions respectives opposées de ces deux branches).

Du point de vue de l'encombrement global du transducteur, cette configuration particulière en deux branches parallèles permet de diminuer dans des proportions importantes la longueur hors-tout du PZT (dimension considérée dans la direction longitudinale de l'axe Δ) par rapport à une lame conventionnelle telle que celle illustrée Figure 3, le gain de longueur étant typiquement d'au moins 20 à 40 %.

D'autre part, avec le double porte-à-faux et les déplacements des deux branches dans des sens contraires, l'amplitude hors-tout de l'oscillation de la lame sera réduite par rapport à un PZT conventionnel du fait que les courses respectives de chacune des deux branches 60, 70 s'additionnent en valeur absolue mais sont en sens contraire. Ceci permet de réduire également dans des proportions importantes le volume nécessaire au débattement de l'ensemble pendulaire par rapport à une configuration conventionnelle, avec une diminution corrélative du volume mort qui, en pratique, est une place perdue qui n'est pas utilisable pour y placer des composants de circuit ou loger une batterie plus volumineuse.

La Figure 7 illustre une variante de la Figure 6 dans laquelle la jonction 80 entre les branches externe et interne 60, 70 n'est pas arrondie, mais rectangulaire. La configuration arrondie de la Figure 6 permet toutefois de mieux répartir les contraintes au niveau de la jonction 80 entre les deux branches, contraintes résultant du fait que ces deux branches fléchissent dans des sens opposés alors même qu'elles sont formées d'une seule pièce.

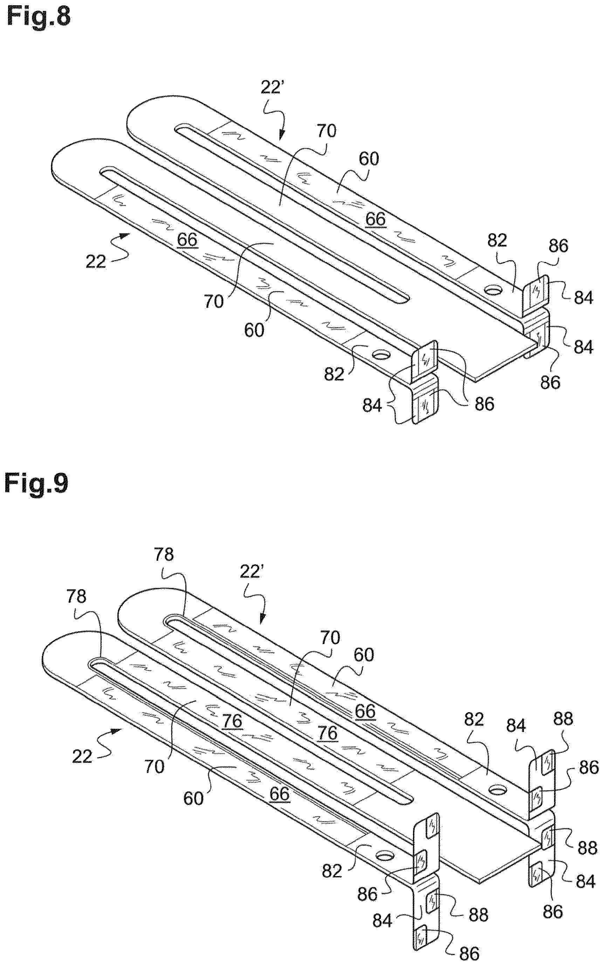

Le recueil des charges électriques produites par les flexions des branches lors des oscillations est assuré par un système d'électrodes illustré sur les vues en perspectives des Figures 8 et 9.

Sur la Figure 8 on a représenté un mode de réalisation dans lequel les branches externes 60 de chaque lame 22, 22' sont munies d'électrodes de surface 66 s'étendant sur la majeure partie de la longueur de la branche. Ces électrodes sont par exemple réalisées par un dépôt d'or, de cuivre, de platine ou de tout autre matière conductrice, et sont reliées électriquement à l'extrémité proximale de la branche à un circuit souple ou *flex* 82 se prolongeant par une partie libre relevée 84 portant une plage de connexion ou *pad* 86 électriquement reliée à l'électrode 66 et permettant le transfert des charges électriques produites par cette dernière vers un circuit électrique d'alimentation.

Si la lame utilisée est une lame bimorphe telle que celle décrite plus haut à propos de la Figure 5, la face inférieure opposée (non visible sur la figure) de la branche externe 60 est munie d'une électrode semblable, reliée à un autre pad 86 à l'extrémité proximale de la même branche. Les électrodes en vis-à-vis sur des faces opposées recueillent alors des charges de signes contraires, conduites aux pads 86 correspondants. L'âme centrale de la lame bimorphe (référencée 50 sur la Figure 5) peut comporter également une liaison électrique à un pad correspondant, utilisée comme potentiel de référence électrique de "neutre".

Avec une lame bimorphe, la configuration de la Figure 8 comporte ainsi au total quatre électrodes 66 (deux électrodes de chaque côté de chaque branche externe 60), quatre flex 82 et quatre pads 86.

Par ailleurs, comme cela est décrit dans la demande EP 20315317.6 précitée, il est possible de fractionner les électrodes de surface, ou certaines d'entre elles, sur la face supérieure et/ou sur la face inférieure de la lame, en deux ou plus sous-électrodes électriquement isolées entre elles. Ces sous-électrodes fractionnaires, qui peuvent être ou non symétriques par rapport à un axe longitudinal central de la lame, sont alors reliées chacune à un pad respectif distinct pour la prise de contact et la liaison à des étages redresseurs en aval.

La Figure 9 illustre un autre mode de réalisation dans lequel, outre les électrodes 66 sur les branches externes 60 de la Figure 8, le PZT comporte également des électrodes 76 sur les branches internes 70.

Dans la mesure où les branches internes 76 se déplacent dans un sens opposé de celui des branches externes 60, les électrodes de surface respectives des branches interne et externe recueilleront des charges de signes opposés au cours d'une même déformation du PZT (charge positive sur les électrodes 66 et charge négative sur les électrodes 76, et *vice versa* à la demi-oscillation suivante). Comme il n'est pas possible pour cette raison de mettre en série les électrodes 76 et 66, chaque électrode 76 est reliée au flex 82 par un conducteur 78 qui court le long de la branche externe 60 en étant électriquement isolé de l'électrode 66, afin d'assurer une prise de connexion par un pad 88 sur la partie libre relevée d'extrémité 84 du flex, au voisinage des pads 86 reliés aux électrodes 66 des branches externes 60.

Avec une configuration d'électrodes semblable sur la face inférieure (non visible sur la figure), on obtient ainsi un agencement comprenant huit électrodes 66, 76, quatre flex 82 et huit pads 86, 88.

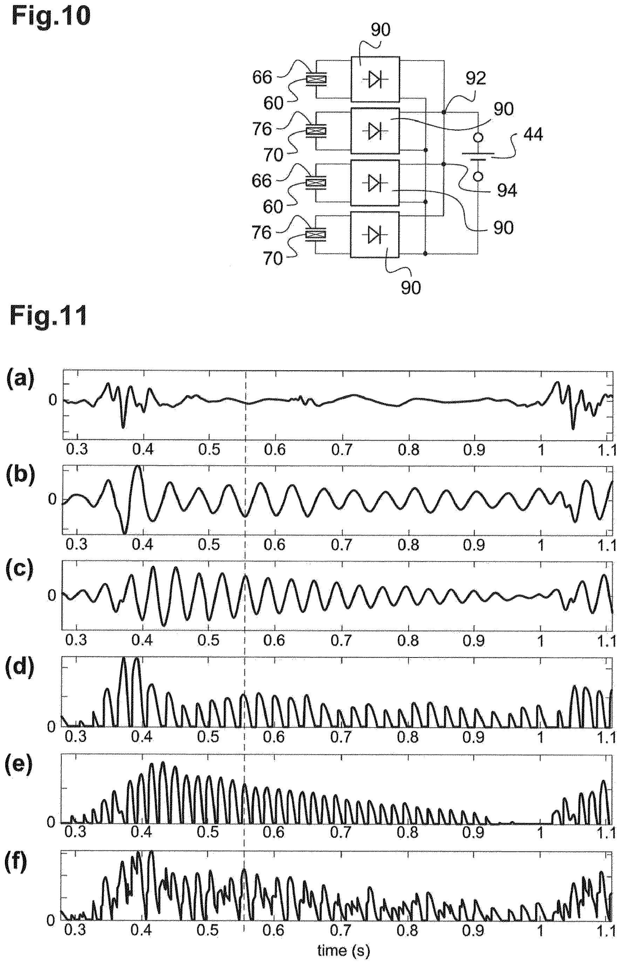

La Figure 10 est un schéma électrique simplifié montrant la manière de coupler entre elles les diverses électrodes de la configuration de la Figure 9 pour délivrer en sortie du PEH un courant d'alimentation et/ou de recharge d'une batterie tampon.

Chaque couple d'électrodes en face inférieure/supérieure de chacune des branches 60 ou 70 est relié à un circuit redresseur respectif 90, par exemple un redresseur double alternance FBR *(Full-Bridge Rectifier),* par exemple un pont de diodes (pont de Graetz), ou un convertisseur de tension négative NVC *(Négative Voltage Converter*) à MOSFET ou autre circuit analogue. Les sorties respectives des circuits redresseurs 90 sont reliées à une borne positive 92 commune et à une borne négative 94 commune délivrant entre elles un courant de recharge appliqué à la batterie tampon 44. Les circuits redresseurs 90 peuvent être éventuellement réalisés avec de composants disposés directement sur les flex 84 et reliés aux pads 86, 88 respectifs.

Avantageusement, les sorties correspondantes (positive ou bien négative) des redresseurs 90 sont directement reliées ensemble, sans couplage intermédiaire.

En variante, il est également possible de relier directement ensemble les diverses électrodes de surface recueillant des charges de même polarité, ces électrodes étant ensuite couplées à une même entrée respective d'un étage redresseur commun, tous les courants générés par le déplacement des charges de même signe s'additionnant pour être délivrés à la batterie après redressement.

La Figure 11 est une série de chronogrammes montrant :
(a) les variations instantanées de l'accélération du PZT ;
(b) le courant généré par une paire d'électrodes supérieure/inférieure de l'une des branches externes 60 ;
(c) le courant généré par une paire d'électrodes supérieure/inférieure de l'une des branches internes 70 ;
(d) le courant (b), après redressement double alternance par un circuit 90 ;
(e) le courant (c), après redressement double alternance par un circuit 90 ; et
(f) le courant total résultant de l'addition des courants (b) et (e), injecté dans la batterie tampon 44 pour la recharge de cette dernière.

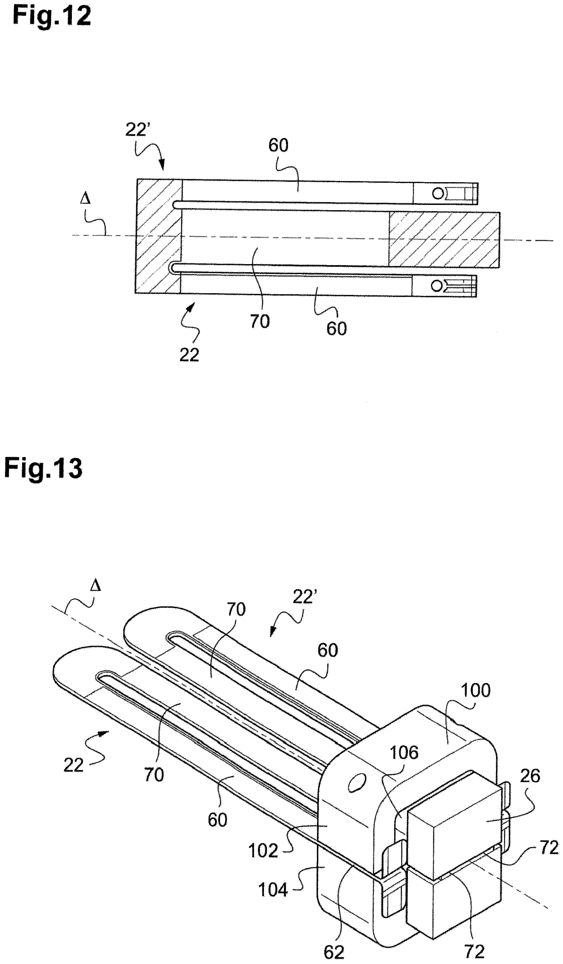

La Figure 12 illustre une variante de réalisation du PZT précédemment décrit en relation aux Figures 6 et 7, dans laquelle les deux branches internes 70 sont confondues en une branche unique sur la totalité de leur longueur (en variante, elles peuvent n'être confondues que sur une partie de leur longueur). Cette variante procure notamment au PZT une rigidité supérieure à l'endroit où se concentrent les contraintes lors des oscillations de l'ensemble pendulaire. En ce qui concerne la configuration des électrodes, la branche interne centrale 70 porte une électrode unique sur chacune des faces supérieure et inférieure, et chacune des deux branches externes 60 porte une électrode sur chacune de ses faces supérieure et inférieure. On dispose dans ce cas d'un ensemble comportant en tout six électrodes, avec six pads et quatre flex.

La Figure 13 illustre le PZT selon l'invention, tel qu'illustré notamment aux Figures 6 et 9, dans une configuration complète de l'ensemble pendulaire, avec les encastrements et la masse inertielle montée.

L'encastrement, au niveau des extrémités proximales 62 de chaque branche externe 60, est réalisé par une monture ou *clamp* 100, constituée ici de deux pièces distinctes 102, 104 assemblées de manière à prendre en sandwich les extrémités proximales 62 des branches externes 60, pour réaliser à cet endroit les encastrements 24 des deux lames 22, 22' du PZT.

La masse inertielle 26 est montée à l'extrémité proximale 72 des branches internes 70, et elle a la possibilité de se déplacer librement dans un espace libre 106 ménagé à cet endroit dans la monture 100, qui présente une forme sensiblement annulaire.

On soulignera que, outre la compacité accrue, cet agencement particulier selon l'invention permet de déporter le centre de gravité G de la masse inertielle 26 en arrière du centre de la monture 100, accroissant d'autant l'effet d'inertie.

Les Figures 14 et 15 montrent en coupe l'ensemble de la Figure 13, respectivement avec le PZT immobile en position centrale (donc en alignement sur l'axe Δ) et en configuration de déformation maximale. Dans ce dernier cas, les branches externes 60 sont déformées dans un premier sens (vers le haut sur la figure) avec une courbure *ρ₁* et une excursion *e₁* par rapport à l'axe Δ, et les branches internes 70 se déforment dans le sens contraire avec une courbure *ρ₂* opposée et une excursion maximale *e₂* par rapport à l'axe Δ. On notera que la déformation totale du PZT correspond à la somme des deux excursions *e₁* et *e₂*, avec l'avantage d'une déformation importante (|*e₁*|+|*e₂*|) dans un relativement faible volume de déplacement, par rapport à une configuration de PZT conventionnelle telle que celle illustrée Figure 3.

Avantageusement, la monture 100 comporte dans le volume interne 106 une surface interne 108 contre laquelle peut venir buter une surface externe en vis-à-vis 114 de la masse inertielle 26, ceci afin d'éviter une déformation excessive du PZT lors de fortes sollicitations et préserver ainsi la longévité de l'ensemble. Cette butée, par contact entre la masse inertielle et la monture, évite tout contact direct avec la lame, comme cela, est le cas dans certains dispositifs de limitation de course connus, par exemple celui décrit par le US 2019/381325 A1 (Regnier et al.).

La Figure 16 illustre une variante de la Figure 15 dans laquelle la surface interne de butée 108 de la monture 100 est une surface inclinée par rapport à l'axe Δ (au lieu d'être parallèle à l'axe Δ comme dans le cas de la Figure 15), et la surface complémentaire 114 de la masse inertielle est une surface plane (au lieu d'être une surface inclinée comme dans le cas de la Figure 15).

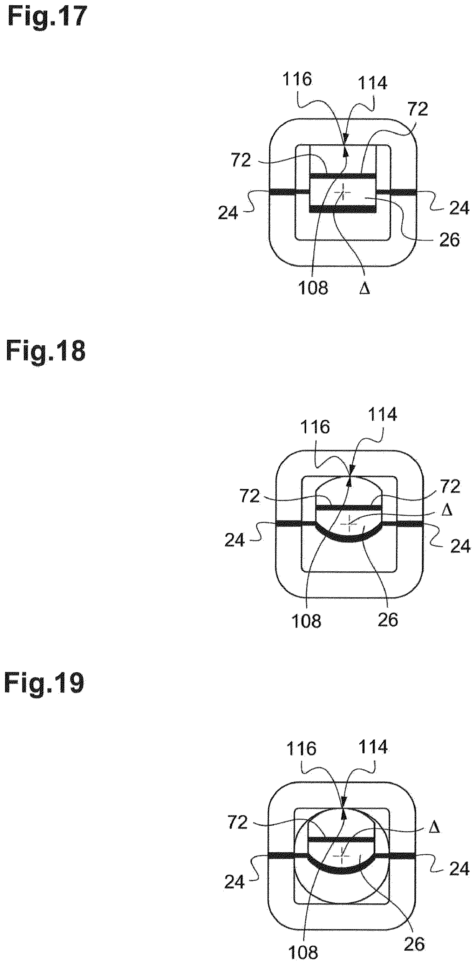

La Figure 17 est une vue en coupe transversale de la monture de la Figure 16, montrant le contact en section droite entre les surfaces complémentaires 108 et 114, qui est un contact plan 116 sur une étendue de surface relativement importante.

Dans une variante illustrée Figure 18, l'une des surfaces 108, 114 est une surface courbe et l'autre une surface plane, et le contact mutuel 118 est un contact linéaire, axial.

Dans un autre variante illustrée Figure 19, les surfaces 108 et 114 sont toutes deux des surfaces courbes, et le contact entre ces surfaces est un contact surfacique-cylindrique, qui procure une meilleure absorption des chocs du fait de la plus grande surface de contact mutuel.

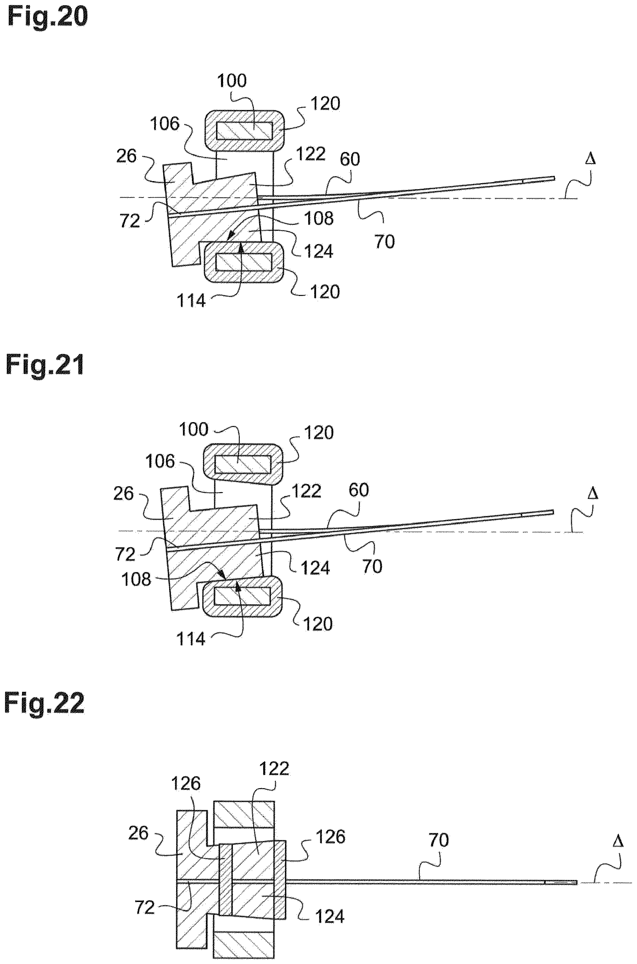

Les Figures 20 et 21 sont homologues des Figures 15 et 16, dans une variante où les zones de contact de la monture 100 sont couvertes d'un revêtement amortisseur approprié, par exemple un polymère ou un silicone, de manière à éviter un contact métal/métal entre la masse inertielle 26 et la monture 100 comme dans les exemples précédents.

La Figure 22 illustre une manière particulière de réaliser le montage de la masse inertielle 26 sur l'extrémité proximale 72 des branches 70.

Dans les exemples précédents, par exemple sur la Figure 20, la masse inertielle 26 est constituée de deux pièces distinctes 122, 124 collées sur l'extrémité 72 des branches internes 70, cette extrémité étant prise en sandwich entre les pièces 122 et 124. Le collage peut toutefois présenter un certain nombre de difficultés et de limitations si l'on veut garantir une fiabilité sans faille du dispositif pendant toute sa durée de vie (au moins 10 ans). Pour ces raisons, une construction non collée peut être préférable, par exemple une construction entièrement soudée telle que celle illustrée Figure 22, où les deux pièces 124, 126 sont réunies ensemble par des goupilles 126 soudées reliant entre elles les pièces 124, 126 en prenant en sandwich l'extrémité proximale 72 des branches internes 70.

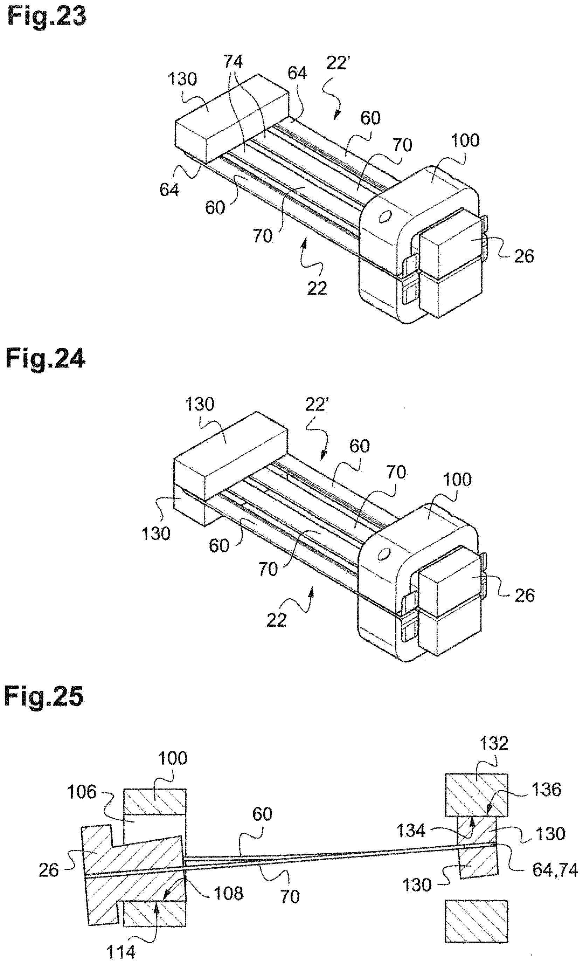

On va maintenant décrire, en référence aux Figures 23 à 28, divers modes de réalisation de l'invention mettant en oeuvre une masselotte intermédiaire 130 lestant les extrémités distales des branches externe et interne 60, 70 à l'endroit de leur jonction 80.

La présence d'une masselotte intermédiaire 130 à cet endroit présente plusieurs avantages :
- il s'agit d'un élément rigide qui supprime les contraintes à l'endroit des courbures 80 de jonction entre des branches internes 70 et externes 60, à un endroit où ces contraintes peuvent être particulièrement élevées ;
- la présence d'une masselotte 130 à cet endroit est sans incidence sur le rendement de conversion, dans la mesure où il s'agit d'une zone de la lame ne produisant pratiquement pas de charges (la flexion du matériau PZT étant minimale à cet endroit, qui ne porte d'ailleurs pas d'électrodes), donc sans incidence sur le rendement électrique du PEH ;
- la masselotte intermédiaire 130 constitue une masse mobile additionnelle pour les branches internes 70 qui accentue l'effet d'oscillation pendulaire pour ces dernières ;
- pour optimiser la production d'énergie par le transducteur, il est possible de dissocier les régimes de vibration des deux lames, comme on l'expliquera en référence aux Figures 26 à 28 ci-après.

Dans la variante de la Figure 23, une masselotte unique 130 est disposée, par exemple collée, sur la face supérieure des deux lames 22, 22' et est commune à ces deux lames.

Dans la variante de la Figure 24, une masselotte 130 est montée sur chacune des deux faces supérieure et inférieure des deux lames 22, 22', les masselottes 130 étant ici encore communes aux deux lames.

La Figure 25 est une vue en coupe de l'ensemble de la Figure 24, dans une configuration de déformation maximale du PZT. Avantageusement, une pièce 132 permet de limiter la déformation maximale du côté des extrémités distales 64, 74 des branches 60, 70, par venue en butée contre une surface interne 134 d'une surface externe homologue 136 de chacune des masselottes intermédiaires 130. Cet agencement assure une double limitation de la déformation du PZT, côté distal par les surfaces de butée 134 de la pièce 132, et côté proximal par les surfaces de butée 108 de la monture 100, à chaque fois sans contact direct avec le matériau de la lame.

Les variantes des Figures 26 et 27 sont homologues de celles des Figures 23 et 24, avec la masselotte intermédiaire 130 fractionnée en deux masselottes distinctes 130, 130', chacune pour l'une des deux lames 22, 22'. Les deux lames ne sont donc pas couplées aux extrémités distales des branches 60, 70, ce qui permet d'en dissocier le. régime d'oscillation.

Les géométries et/ou les masses et/ou les masses volumiques des masselottes intermédiaires 130, 130' peuvent être choisies de manière à produire des régimes d'oscillation différents pour chacune des deux lames 22, 22', pour optimiser la récupération d'énergie. De plus, outre les masselottes intermédiaires 130, 130', il est possible de donner aux deux lames des longueurs et/ou des largeurs différentes pour modifier leur mode de vibration, par exemple des longueurs différentes des branches comme cela est illustré avec les lames 22, 22' sur la Figure 28.

## Revendications

1. Un module de récupération d'énergie comprenant un ensemble pendulaire soumis à des sollicitations externes appliquées au module,
l'ensemble pendulaire comprenant un transducteur piézoélectrique s'étendant, le long d'un axe central (Δ) correspondant à une direction de plus grande longueur du transducteur piézoélectrique, d'une extrémité distale à une extrémité proximale opposée, le transducteur étant élastiquement déformable en flexion entre une extrémité encastrée (24) et une extrémité libre couplée à une masse inertielle (26),
le transducteur piézoélectrique étant apte à convertir une énergie mécanique produite par des oscillations de l'ensemble pendulaire en un signal électrique oscillant (V_{OUT}(t)) recueilli par des électrodes de surface du transducteur piézoélectrique,
dans lequel:
- le transducteur piézoélectrique comprend deux lames piézoélectriques (22, 22') coplanaires agencées côte-à-côte de part et d'autre dudit axe central (Δ), chacune des lames piézoélectriques comprenant une branche externe (60) et une branche interne (70) attenantes agencées côte-à-côte et formées d'un seule pièce,
- la branche externe (60) de chaque lame piézoélectrique a une extrémité proximale (62) encastrée (24) et une extrémité distale (64) libre,
- la branche interne (70) de chaque lame piézoélectrique a une extrémité proximale (72) libre et supportant la masse inertielle (26), et une extrémité distale (74) libre et réunie à l'extrémité distale (64) de la branche externe (60) attenante par une jonction (80) commune, et
- la masse inertielle (26) ainsi que les extrémités proximales (62) encastrées respectives (24) des branches externes (60) des deux lames piézoélectriques (22, 22') sont agencées à l'extrémité proximale du transducteur piézoélectrique, et les jonctions communes respectives (80) des deux lames piézoélectriques (22, 22') sont agencées à l'extrémité distale du transducteur piézoélectrique,
de sorte que les oscillations de l'ensemble pendulaire produisent des déplacements respectifs des deux branches internes (70) dans un premier sens et, simultanément, des deux branches externes (60) dans un second sens opposé.

2. Le module de la revendication 1, dans lequel il est prévu des électrodes de surface (66, 76) sur l'une au moins des branches externe (60) et interne (70) de chaque lame piézoélectrique, et dans lequel le module comprend en outre un étage redresseur (90) recevant sur des entrées respectives les charges recueillies par les électrodes de surface de chaque lame piézoélectrique et délivrant en sortie une signal d'alimentation redressé.

3. Le module de la revendication 2, dans lequel il est prévu des électrodes de surface (66, 76) sur l'une et l'autre des branches externe (60) et interne (70) de chaque lame piézoélectrique, les électrodes de surface respectives des branches interne et externe étant aptes à recueillir des charges de polarités opposées au cours d'une même déformation du transducteur piézoélectrique.

4. Le module de la revendication 3, dans lequel les électrodes de surface recueillant des charges de même polarité sont directement reliées ensemble et couplées à une même entrée respective de l'étage redresseur.

5. Le module de la revendication 2 ou 3, dans lequel les lames piézoélectriques sont des lames bimorphes comprenant une âme centrale (50) conductrice formant électrode commune et, pour chaque branche interne et/ou externe, des électrodes de surface (52, 54) sur une face supérieure et sur une face inférieure.

6. Le module de la revendication 4 ou 5, dans lequel l'étage redresseur comprend une pluralité de circuits redresseurs (90) associés à des paires d'électrodes respectives délivrant des charges de polarités opposées, et dans lequel les sorties de la pluralité de circuits redresseurs sont directement reliées ensemble.

7. Le module de la revendication 1, dans lequel les branches externe (60) et interne (70) de chaque lame piézoélectrique sont parallèles et/ou rectilignes et/ou sont de même longueur pour les deux lames piézoélectriques.

8. Le module de la revendication 1, dans lequel les branches interne et externe sont de longueurs différentes pour les deux lames piézoélectriques respectives.

9. Le module de la revendication 1, dans lequel les branches internes respectives des deux lames piézoélectriques sont partiellement ou totalement confondues en une branche unique (70).

10. Le module de la revendication 1, dans lequel l'une au moins des lames piézoélectriques (22, 22') porte à son extrémité distale libre une masselotte intermédiaire (130 ; 130') à l'endroit de la jonction (80) des branches interne (70) et externe (60).

11. Le module de la revendication 10, dans lequel les deux lames piézoélectriques portent une masselotte intermédiaire commune (130) à l'endroit de la jonction.

12. Le module de la revendication 10, dans lequel les deux lames piézoélectriques portent chacune indépendamment une masselotte propre (130, 130') à l'endroit de la jonction.

13. Un dispositif autonome logeant, dans un corps de dispositif :
- un ensemble électronique (28-38) ;
- un module de récupération d'énergie (40) selon l'une des revendications précédentes, produisant en sortie un signal électrique oscillant ;
- un circuit de gestion d'alimentation (42), apte à redresser et réguler le signal électrique oscillant produit par le module de récupération d'énergie, pour délivrer en sortie une tension ou un courant continus d'alimentation stabilisés ; et
- un organe de stockage d'énergie (44) pour l'alimentation de l'ensemble électronique,
dans lequel ladite tension ou ledit courant continus stabilisés délivrés par le circuit de gestion d'alimentation servent à l'alimentation de l'ensemble électronique et/ou à la recharge de l'organe de stockage d'énergie du dispositif autonome.

14. Le dispositif autonome de la revendication 13,
dans lequel le dispositif autonome est un dispositif médical actif de type capsule autonome implantable (10) comprenant un corps de capsule (12) avec un élément d'ancrage (16) à une paroi d'un organe d'un patient,
et dans lequel les sollicitations externes auxquelles est soumis l'ensemble pendulaire (22, 26) du module de récupération d'énergie sont des sollicitations appliquées au corps de capsule (12) sous l'effet de mouvements de ladite paroi et/ou de variations de débit d'un flux dans le milieu environnant.

## Patentansprüche

1. Ein Energierückgewinnungsmodul, umfassend eine pendelartige Anordnung, die äußere Belastungen erfährt, die auf das Modul aufgebracht werden,
wobei die pendelartige Anordnung einen piezoelektrischen Wandler umfasst, der sich, entlang einer mittigen Achse (Δ), die einer Richtung der größten Länge des piezoelektrischen Wandlers entspricht, von einem distalen Ende zu einem gegenüberliegenden proximalen Ende erstreckt, wobei der Wandler zwischen einem eingespannten Ende (24) und einem freien Ende, das an eine träge Masse (26) gekoppelt ist, elastisch biegeverformbar ist,
wobei der piezoelektrische Wandler imstande ist, eine mechanische Energie, die durch Schwingungen der pendelartigen Anordnung erzeugt wird, in ein elektrisches Schwingungssignal (V_{OUT}(t)) umzuwandeln, das von Oberflächenelektroden des piezoelektrischen Wandlers empfangen wird,
wobei:
- der piezoelektrische Wandler zwei koplanare piezoelektrische Längsorgane (22, 22') umfasst, die nebeneinander auf beiden Seiten der mittigen Achse (Δ) angeordnet sind, wobei jedes der piezoelektrischen Längsorgane einen äußeren Schenkel (60) und einen inneren Schenkel (70) umfasst, die sich anschließen, nebeneinander angeordnet und aus einem Stück gebildet sind,
- der äußere Schenkel (60) jedes piezoelektrischen Längsorgans ein eingespanntes (24) proximales Ende (62) und ein freies distales Ende (64) aufweist,
- der innere Schenkel (70) jedes piezoelektrischen Längsorgans ein freies proximales Ende (72), das die inertiale Masse (26) trägt, und ein freies distales Ende (74), das mit dem distalen Ende (64) des äußeren Schenkels (60), der sich über eine gemeinsame Verbindungsstelle (80) anschließt, verbunden ist, aufweist und
- die inertiale Masse (26) sowie die jeweiligen eingespannten (24) proximalen Enden (62) der äußeren Schenkel (60) der zwei piezoelektrischen Längsorgane (22, 22') an dem proximalen Ende des piezoelektrischen Wandlers angeordnet sind und die jeweiligen gemeinsamen Verbindungen (80) der zwei piezoelektrischen Längsorgane (22, 22') an dem distalen Ende des piezoelektrischen Wandlers angeordnet sind,
so dass die Schwingungen der pendelartigen Anordnung jeweilige Bewegungen der zwei inneren Schenkel (70) in einer ersten Richtung und, gleichzeitig, der zwei äußeren Schenkel (60) in einer entgegengesetzten zweiten Richtung bewirken.

2. Das Modul nach Anspruch 1, wobei Oberflächenelektroden (66, 76) auf dem wenigstens einen der Schenkel, dem äußeren (60) und dem inneren (70), jedes piezoelektrischen Längsorgans vorgesehen sind und wobei das Modul ferner eine Gleichrichterstufe (90) umfasst, die an jeweiligen Eingängen die von den Oberflächenelektroden jedes piezoelektrischen Längsorgans empfangenen Lasten aufnimmt und ausgangsseitig ein gleichgerichtetes Versorgungssignal ausgibt.

3. Das Modul nach Anspruch 2, wobei Oberflächenelektroden (66, 76) auf dem wenigstens einen der Schenkel, dem äußeren (60) und dem inneren (70), jedes piezoelektrischen Längsorgans vorgesehen sind, wobei die jeweiligen Oberflächenelektroden des inneren Schenkels und des äußeren Schenkels imstande sind, Lasten entgegengesetzter Polaritäten im Laufe einer selben Verformung des piezoelektrischen Wandlers aufzunehmen.

4. Das Modul nach Anspruch 3, wobei die Oberflächenelektroden, die Lasten gleicher Polarität aufnehmen, direkt miteinander verbunden und mit einem jeweiligen gleichen Eingang der GleichrichterStufe gekoppelt sind.

5. Das Modul nach Anspruch 2 oder 3, wobei die piezoelektrischen Längsorgane bimorphe Längsorgane sind, die eine leitende mittige Seele (50), die eine gemeinsame Elektrode bildet, und, für jeden inneren und/oder äußeren Schenkel, Oberflächenelektroden (52, 54) auf einer oberen Seite und auf einer unteren Seite umfassen.

6. Das Modul nach Anspruch 4 oder 5, wobei die Gleichrichterstufe eine Mehrzahl von Gleichrichterschaltungen (90) umfasst, die jeweiligen Elektrodenpaaren, die Lasten entgegengesetzter Polaritäten bereitstellen, zugeordnet sind, und wobei die Ausgänge der Mehrzahl von Gleichrichterschaltungen direkt miteinander verbunden sind.

7. Das Modul nach Anspruch 1, wobei der äußere Schenkel (60) und der innere Schenkel (70) jedes piezoelektrischen Längsorgans parallel und/oder geradlinig sind und/oder bei den zwei piezoelektrischen Längsorganen gleicher Länge sind.

8. Das Modul nach Anspruch 1, wobei der innere und der äußere Schenkel bei den jeweiligen zwei piezoelektrischen Längsorganen verschiedener Längen sind.

9. Das Modul nach Anspruch 1, wobei die jeweiligen inneren Schenkel der zwei piezoelektrischen Längsorganen zum Teil oder zur Gänze in einen einzigen Schenkel (70) übergehen.

10. Das Modul nach Anspruch 1, wobei das wenigstens eine der piezoelektrischen Längsorgane (22, 22') an seinem freien distalen Ende ein dazwischen angeordnetes Pendelgewicht (130; 130') an der Stelle der Verbindung (80) des inneren Schenkel (70) und des äußeren Schenkels (60) trägt.

11. Das Modul nach Anspruch 10, wobei die zwei piezoelektrischen Längsorgane ein gemeinsames dazwischen angeordnetes Pendelgewicht (130) an der Stelle der Verbindung tragen.

12. Das Modul nach Anspruch 10, wobei die zwei piezoelektrischen Längsorgane jedes unabhängig ein eigenes Pendelgewicht (130, 130') an der Stelle der Verbindung tragen.

13. Eine autonome Vorrichtung, die in einem Vorrichtungskörper Folgendes aufnimmt:
- eine elektronische Anordnung (28-38);
- ein Energierückgewinnungsmodul (40) nach einem der vorhergehenden Ansprüche, das ausgangsseitig ein elektrisches Schwingungssignal erzeugt;
- eine Versorgungsverwaltungsschaltung (42), die imstande ist, das von dem Energierückgewinnungsmodul erzeugte elektrische Schwingungssignal gleichzurichten und zu regeln, um ausgangsseitig eine stabilisierte Vorsorgungsgleichspannung oder einen stabilisierten Versorgungsgleichstrom bereitzustellen; und
- ein Energiespeicherorgan (44) für die Versorgung der elektronischen Anordnung,
wobei die stabilisierte Gleichspannung oder der stabilisierte Gleichstrom, die/der von der Versorgungsverwaltungsschaltung bereitgestellt wird, der Versorgung der elektronischen Anordnung und/oder der Aufladung des Energiespeicherorgans der autonomen Vorrichtung dienen.

14. Die autonome Vorrichtung nach Anspruch 13, wobei die autonome Vorrichtung eine aktive medizinische Vorrichtung des Typs implantierbare autonome Kapsel (10) ist, die einen Kapselkörper (12) mit einem Element (16) zur Verankerung an einer Wand eines Organs eines Patienten umfasst, und wobei die äußeren Belastungen, die die pendelartige Anordnung (22, 26) des Energierückgewinnungsmoduls erfährt, Belastungen sind, die auf den Kapselkörper (12) unter der Einwirkung von Bewegungen der Wand und/oder von Veränderungen einer Blutflussrate im umgebenden Medium aufgebracht werden.

## Claims

1. An energy harvesting module, comprising a pendular unit subjected to external stresses applied to the module,
the pendular unit comprising a piezoelectric transducer extending, along a central axis (Δ) corresponding to a direction of greater length of the piezoelectric transducer, from a distal end to an opposite proximal end, the transducer being elastically deformable in bending between a clamped end (24) and a free end coupled to an inertial mass (26),
the piezoelectric transducer being adapted to convert a mechanical energy produced by oscillations of the pendular unit into an oscillating electrical signal (V_{OUT}(t)) collected by surface electrodes of the piezoelectric transducer,
wherein:
- the piezoelectric transducer comprises two coplanar piezoelectric beams (22, 22') arranged side-by-side on either side of said central axis (Δ), each of the piezoelectric beams comprising adjacent external (60) and internal (70) arms, arranged side-by-side and formed single-piece,
- the external arm (60) of each piezoelectric beam has a clamped (24) proximal end (62) and a free distal end (64),
- the internal arm (70) of each piezoelectric beam has a free proximal end (72) supporting the inertial mass (26), and a free distal end (74) connected to the distal end (64) of the adjacent external arm (60) by a common junction (80), and
- the inertial mass (26) as well as the respective clamped (24) proximal ends (62) of the external arms (60) of the two piezoelectric beams(22, 22') are arranged at the proximal end of the piezoelectric transducer, and the respective common junctions (80) of the two piezoelectric beams (22, 22') are arranged at the distal end of the piezoelectric transducer,
whereby the oscillations of the pendular unit produce respective displacements of the two internal arms in a first direction and, simultaneously, of the two external arms in a second, opposite direction.

2. The module of claim 1, wherein surface electrodes (66, 76) are provided on one at least of the external (60) and internal (70) arms of each piezoelectric beam, and wherein the module further comprises a rectifier stage (90) receiving on respective inputs the charges collected by the surface electrodes of each piezoelectric beam and outputting a rectified power supply signal.

3. The module of claim 2, wherein surface electrodes (66, 76) are provided on either of the external (60) and internal (70) arms of each piezoelectric beam, the respective surface electrodes of the internal and external arms being adapted to collect charges of opposed polarities during a same deformation of the piezoelectric transducer.

4. The module of claim 3, wherein the surface electrodes collecting charges of same polarity are directly connected to each other and coupled to a same respective input of the rectifier stage.

5. The module of claim 2 or 3, wherein the piezoelectric beams are bimorphous beams comprising a conductive central core (50) forming a common electrode and, for each internal and/or external arm, surface electrodes (52, 54) on a top and a bottom face.

6. The module of claim 4 or 5, wherein the rectifier stage comprises a plurality of rectifier circuits (90) associated with respective pairs of electrodes outputting charges of opposed polarities, and the outputs of the plurality of rectifier circuits are directly connected to each other.

7. The module of claim 1, wherein the external (60) and internal (70) arms of each piezoelectric beam are parallel and/or rectilinear and/or are of same length for both piezoelectric beams.

8. The module of claim 1, wherein the internal and external arms are of different lengths for the two respective piezoelectric beams.

9. The module of claim 1, wherein the respective internal arms of the two piezoelectric beams are partially or fully merged into a single arm (70).

10. The module of claim 1, wherein one at least of the piezoelectric beams (22, 22') carries at its free distal end an intermediate flyweight (130 ; 130') at the junction (80) between the internal (70) and external (60) arms.

11. The module of claim 10, wherein the two piezoelectric beams carry a common intermediate flyweight (130) at the junction.

12. The module of claim 10, wherein the two piezoelectric beams each carry independently their own flyweight (130, 130') at the junction.

13. An autonomous device, housing within a device body:
- an electronic unit (28-38);
- an energy harvesting module (40) according to any one of the previous claims, outputting an oscillating electric signal;
- a power management circuit (42), adapted to rectify and regulate the oscillating electric signal produced by the energy harvesting module, to output stabilized direct voltage or current; and
- an energy storage component (44) for powering the electronic unit,
wherein said stabilized direct voltage or current provided by the power management circuit is used to power the electronic unit and/or to charge the energy storage component of the autonomous device.

14. The autonomous device of claim 13,
wherein the autonomous device is an active medical device of the implantable autonomous capsule (10) type, comprising a capsule body (12) with an element (16) for its anchoring to a wall of a patient's organ,
and wherein the external stresses to which is subjected the pendular unit (22, 26) of the energy harvesting module are stresses applied to the capsule body (12) under the effect of movements of said wall and/or blood flow rate variations in a surrounding environment.
